Europäisches Patentamt

European Patent Office

Office européen des brevets

(19)

(11) Publication number: **0 119 650**
**A2**

(12) **EUROPEAN PATENT APPLICATION**

(21) Application number: **84200328.7**

(22) Date of filing: **08.03.84**

(51) Int. Cl.³: **A 61 K 37/26**

(30) Priority: **21.03.83 US 477555**

(43) Date of publication of application: **26.09.84**
**Bulletin 84/39**

(84) Designated Contracting States: **AT BE CH DE FR GB IT LI LU NL SE**

(71) Applicant: **THE PROCTER & GAMBLE COMPANY, 301 East Sixth Street, Cincinnati Ohio 45202 (US)**

(72) Inventor: **Myhre, David Vernon, 220 Brocdorf Drive, Cincinnati Ohio 45215 (US)**
Inventor: **Geho, Walter Blair, c/o Technology Ltd., Inc. 1273 Lincoln Way West, Wooster Ohio 44691 (US)**

(74) Representative: **Suslic, Lydia et al, Procter & Gamble European Technical Center Temselaan 100, B-1820 Strombeek-Bever (BE)**

(54) Galactosyl-insulin conjugates useful in treating diabetics.

(57) Biologically active galactosyl-insulin conjugates useful in the treatment of insulin-dependent diabetics, in particular diabetic humans are disclosed herein. The conjugates comprise at least one D-galactosyl group covalently bonded to insulin. The D-galactosyl group targets the conjugates to the hepatocytes. When these conjugates are administered to the diabetic, a more effective amount of insulin can be delivered to the liver for control of blood glucose levels.

EP 0 119 650 A2

ACTORUM AG

## GALACTOSYL-INSULIN CONJUGATES USEFUL
## IN TREATING DIABETICS

David V. Myhre

Walter B. Geho

### TECHNICAL FIELD

The present invention relates to targeted insulin delivery systems for use in the treatment of insulin-dependent diabetics, in particular diabetic humans.

Insulin is a hormone which affects the overall metabolism of humans, the most observable effect being the reduction of the concentration of glucose in blood. Ingested carbohydrates are normally broken down to simple sugars in the gut and then absorbed into the portal circulation. The pancreas responds to this sugar load with a release of insulin into the portal circulation which carries the absorbed sugars and the released insulin to the liver. The insulin receptors on the hepatocyte cells bind most of the insulin to regulate the metabolism and synthesis of glucose in the liver cells. Some of the insulin passes on to peripheral body tissues to facilitate glucose uptake by muscle amd adipose tissue. The total reduction of blood glucose by insulin is due to such effects both in the liver and peripheral tissues.

Diabetes mellitus is a generalized, chronic metabolic disorder which can lead to progressive degeneration of the kidneys, the nervous system, and the blood vessels in the eye and the extremities. In many diabetics, the pancreas either produces or releases insufficient insulin. In others, adequate levels of insulin are produced or released, but are not effectively handled by the body, either due to some defect in the insulin itself, or to a resistance the individual develops to the endogenous insulin. In either event, there is a lack of usable insulin so that most tissues cannot metabolize glucose in the blood, eventually resulting in a severe hyperglycemic state. In addition, the tissues of the body are unable to metabolize other nutrients properly; growth is usually impaired. If left untreated, the lack of insulin in these tissues eventually results in abnormally high levels of toxic

0119650

- 2 -

ketone metabolites in the blood. The final combined effect can be coma and death.

For insulin deficient diabetic individuals, insulin administration becomes critical. In current medical practice, insulin is injected subcutaneously because oral administration is inefficient, presumably due to proteolysis. Subcutaneously administered insulin does produce a lowering of the blood glucose level. However, administration of insulin by injection cannot be considered physiologically equivalent to the pancreas producing insulin in a non-diabetic individual. When injected, the peripheral tissues have first access to the insulin, the exact opposite of a non-diabetic individual. The peripheral tissues absorb this injected insulin which reduces the level of insulin reaching the liver and thus decreases the effectiveness of the liver as a significant glucose regulating mechanism. If significant amounts of insulin are injected to achieve proper hepatic control, the peripheral tissues are then overregulated. Therefore, insulin administered by injection does not provide the same physiological action as insulin released from the pancreas of a non-diabetic individual.

One method for overcoming these problems is to specifically target the insulin to the liver. One criterion of such a targeted delivery system is retention of significant biological activity by the insulin molecule. See U.S. Patent 4,003,792 to Mill et al., issued January 18, 1977 (non-targeted conjugates of polysaccharides such as pectin and polypeptides such as insulin which remain biologically active; conjugates of alginic acid and insulin specifically disclosed); U.S. Patent 3,645,852 to Axen et al., issued February 29, 1972 (method of binding water insoluble polysaccharides or derivatives thereof such as dextran to water soluble peptides such as insulin using cyanogen halide to form non-targeted conjugates which retain biological activity). Another desirable feature is the ability to prepare the targeted insulin delivery system by an easy, economical method. Also, the targeted system should preferably remain stable over time.

One such targeted insulin delivery system is disclosed in commonly assigned European Patent Application 9,842 to Geho, published April 16, 1980. Insulin is encapsulated within a lipid membrane structure which comprises a major portion of a polar lipid, preferably distearoyl lecithin, and a minor portion of a digalactosyl derivative, preferably digalactosyl diglyceride, to form an insulin-containing vesicle. The digalactosyl derivative acts as the liver targeting agent for this insulin-containing vesicle. Such vesicles are basically formed by mixing together the polar lipid and the digalactosyl derivative, adding the insulin and then sonicating the resultant mixture. See also European Patent Application 28,917 to Merck & Co., Inc. published May 20, 1981, which discloses lipid vesicles in which the bilayer wall includes cholesterol and distearoyl lecithin together with various cholesterol derivatives or hydrocarbons with charged hydrophilic groups which controllably release drugs or other beneficial agents (e.g. hormones) carried by or within the vesicle, and which discloses that certain aminogalactose derivatives of cholesterol have the characteristic of targeting the vesicles to various parts of the body.

Insulin-containing vesicles provide a viable method for delivering insulin to the liver. The biological activity of the insulin contained within the vesicle is significant, undoubtedly due to the fact that the insulin molecule is left chemically unmodified. However, the preparation of insulin-containing vesicles, especially by the sonication method disclosed in the Geho Application, can be difficult to control. In particular, the yield of insulin-containing vesicles can be extremely low, necessitating a difficult separation step for reuse of insulin which may or may not retain its biological activity. Moreover, there is a tendency of the membrane structure of the vesicle to disintegrate over time, thus releasing the insulin so that the targeted insulin delivery system is destroyed. While insulin-containing vesicles satisfy the criteria of biological activity, the difficulty of preparation and the potential lack of stability over time makes alternative targeted insulin delivery systems attractive.

It is therefore an object of the present invention to provide a targeted insulin delivery system for the liver which does not require vesicles.

It is another object of the present invention to provide a targeted delivery system which provides biologically active insulin to the liver.

It is a further object of the present invention to provide a targeted insulin delivery system which can be prepared by conventional synthetic methods.

It is yet a further object of the present invention to provide a targeted insulin delivery system which is relatively stable over time.

These and further objects of the present invention are hereinafter disclosed.

## OTHER BACKGROUND ART

Lea et al., "Reaction Between Proteins and Reducing Sugars in the Dry State: Reactions of D-galactose, 2-Deoxy-D-galactose, D-glucosamine, and N-acetyl-D-glucosamine with Casein," Biochim. et. Biophys. Acta., Vol. 9, (1952), pp. 56-60 (Chem. Abs. 47:6452d), discloses the reactions of D-galactose, 2-deoxy-D-galactose, D-glucosamine and N-acetyl-D-glucosamine with casein.

U.S. Patent 3,847,890 to Green et al., issued November 12, 1974 discloses conjugates having a reduced antigenic response formed by reacting insulin with an acidic monosaccharide, in particular N-acetyl neuraminic acid or gluconic acid.

Roth et al., "Insulin-Ricin B Chain Conjugate: A Hybrid Molecule with Ricin-Binding Activity and Insulin Biological Activity," J. Biol. Chem., Vol. 256, No. 11, (1981), pp. 5350-54, discloses an insulin-ricin B chain conjugate which binds to the insulin receptors and has a potency one-twentieth that of native insulin.

Insulin-dextran complexes and conjugates are also known. See U.S. Patent 3,788,948 to Kagedal et al., issued January 29, 1974; Kagedal et al., "Binding of Covalent Proteins to Polysaccharides by Cyanogen Bromide and Organic Cyanates: Preparation of Soluble Glycine-, Insulin- and Ampicillin-dextran," Acta.

0119650

- 5 -

Chem. Scand., Vol. 25, (1971), pp. 1855-59; Japanese Patent Document 80-8,916 to Meito Sangyo Co., Ltd., published February 3, 1973; Japanese Patent Document 48-4,623 to Meito Sangyo Co., Ltd., published January 20, 1973; Belgian Patent 813,251 to Zambeletti SPA, published July 31, 1974; Tarui et al. "Parallel Stimulation of Sugar Transport and Glycogen Formation by a Synthetic Insulin-Dextran Complex in Diaphragms," Endocrinology, Vol. 91, (1972), pp. 1442-46 (Chem. Abs. 75:53012x); Suzuki et al., "Mode of Action of Insulin: Properties and Biological Activity of an Insulin-Dextran Complex," Endocrinology, Vol. 90, (1972), pp. 1220-30 (Chem. Abs. 77:56944t); Garwin et al., "Induction of Tyrosine Aminotransferase by Sepharose-Insulin," Arch. Biochem. Biophys., Vol. 164, (1974), pp. 52-59 (Chem. Abs. 81:163686q); Armstrong et al. "Dextran-Linked Insulin: Soluble High Molecular Weight Derivative with Biological Activity in Vivo and In Vitro," Biochem. Biophys. Res. Commun., Vol. 47, (1972), p. 354-60 (Chem. Abs. 77:56927q); Barker et al., "Reaction of Dextran Carbonate with Amino Acids and Polypeptides," Carbohyd. Res., Vol. 25, (1972), pp. 237-41 (Chem. Abs. 78:58780f); Topper et al., "Characterization of Super-Active Insulin, Prolactin and Placental Lactogen," Biochem. Biophys, Res. Commun., Vol. 66, (1975), pp. 793-98 (Chem. Abs. 84:510a); Topper et al. "Insulin and the Mammary Epithelial Cell Membrane," Role. Membranes Metab. Regul. Proc. Symp., (1972), pp. 341-47 (Chem. Abs. 78:119568s); Kolb et al., "Reevaluation of Sepharose-Insulih as a Tool for the Study of Insulin Action," Proc. Natl. Acad. Sci. U.S.A., Vol. 72, (1975), pp. 248-53 (Chem. Abs. 82:106709w); Sakamoto et al., "Comparative Effects of Native Insulin and Insulin-Dextran Complexes on the Metabolism of Adipose Tissue," Biochim. Biophys. Acta., Vol. 498, (1977), pp. 102-13 (Chem. Abs 87:96338m); Cuatrecasus, "Interaction of Insulin with the Cell Membrane: Primary Action of Insulin," Proc. Nat. Acad. Sci. U.S., Vol. 63, (1969), pp. 450-57 (Chem. Abs. 71:88160b); Molteni, "Method of Prolonging the Activity of Drugs by Formation of Macromolecular Compounds," Eur. J. Med. Chem-Chin. Ther., Vol. 9, (1974), pp. 618-20 (Chem. Abs. 83:48130h).

Glucose-insulin conjugates are also known. See Tannenbaum, "Protein Carbonyl Browning Systems: A Study of the Reaction Between Glucose and Insulin," J. Food Sci., Vol. 37, (1966), pp. 53-57; Schwartz et al., "The Reaction Between Proteins and Reducing Sugars in the 'Dry' State: Relative Reactivity of the Alpha- and Epsilon- Amino Groups of Insulin," Biochem. J., Vol. 50, (1952), pp. 713-16; Anzenbacher et al., "Binding of D-Glucose to Insulin," Biochim. Biophys. Acta., Vol. 386, (1975), pp. 603-7 (Chem. Abs. 83:1320e); Clark et al., "Isolation and Characterization of Pigments from Protein-Carbonyl Browning Systems: Models for Two Insulin-Glucose Pigments," J. Agric. Food Chem., Vol. 22, (1974), pp. 1089-93 (Chem. Abs. 87:147206c).

V. Uexkul, "Galactose and Insulin," Z. F. Klin. Medizin, Vol. 140, (1942), pp. 327-42, relates to a study of galactose metabolism, especially by insulin.

Various nonsaccharide-insulin conjugates are known, many of which are useful as hypoglycemic agents. See U.S. Patent 3,823,121 to Grant et al., issued July 9, 1974 (N-aminoacyl substituted insulins); German Patent Document 2,600,971 to Nordisk Insulinlab, published July 22, 1976 (low-allergenic insulin conjugates prepared by reaction of insulin with organic bases); Belgian Patent 770,758 to Farbwerke Hoechs, issued January 1, 1972 (acyl derivatives of insulin); German Patent Document 2,162,164 to Farbwerke Hoechst, published March 15, 1973 (bis(t-butyloxycarbonyl) insulin conjugates); U.S. Patent 3,528,960 to Haas, issued September 15, 1970 (N-carboxyaroyl-insulin conjugates).

## DISCLOSURE OF THE INVENTION

The present invention relates to biologically active galactosyl-insulin conjugates useful in the treatment of insulin-dependent diabetics, in particular diabetic humans. These conjugates comprise at least one D-galactosyl group covalently bonded to insulin. The D-galactosyl moiety targets the conjugate to the hepatocytes so that a better balance of insulin can be delivered to the liver and peripheral tissues for control of blood glucose levels in the diabetic. By targeting the insulin to the liver, the conjugates of the present invention permit a closer approximation of the insulin release mechanism of a non-diabetic wherein insulin produced by the pancreas is initially delivered to the liver.

As previously noted in the Technological Field and Background Art sections, numerous biologically active complexes and conjugates between insulin and a variety of saccharide or saccharide-like molecules are known in the art. However, the key to the present invention lies in the covalent attachment to insulin of a particular saccharide molecule, namely a D-galactosyl group, and the use of the conjugate formed to target biologically active insulin to the liver. It has been determined that the asialoglycoprotein receptors of the hepatocytes exhibit a high degree of preference for D-galactosyl groups, especially N-acetyl-D-galactosylamine. It has also been found that these hepatocytes exhibit a strong affinity for insulin molecules to which such D-galactosyl groups have been covalently attached. Thus, the galactosyl-insulin conjugates of the present invention are specifically targeted for delivery to the liver.

The galactosyl-insulin conjugates of the present invention are particularly good at satisfying the requirements of a targeted insulin delivery system. The biological activity of the insulin molecules of the conjugates is significant relative to native insulin. In fact, because of the liver targeting property, the conjugates of the present invention are effective (i.e. the hepatic effect as measured in diabetic dogs) at much lower dosages than injected native insulin. These conjugates can also be easily prepared by known synthetic procedures for coupling other saccharides to insulin. Moreover, these conjugates are relatively stable as to biological activity and liver targeting ability over time.

## A. Galactosyl-Insulin Conjugates

The biologically active component of the galactosyl-insulin conjugates of the present invention is the insulin molecule. As used herein, the term "biologically active" refers to the ability of the insulin molecule to control, in particular to reduce, the blood glucose levels of the diabetic. The insulin molecule of the conjugates of the present invention have significant biological activity relative to native, chemically unbonded, insulin. Typically, the biological activity (peripheral effect as measured in nondiabetic mice) of these conjugates is about 70-75% that of native insulin.

As used herein, the term "insulin" refers to any insulin molecule which is capable of forming galactosyl-insulin conjugates according to the present invention and which is biologically active when administered to a diabetic. Examples of suitable insulin include human, bovine and porcine insulin. The particular source of insulin is not especially important to the practice of the present invention. Thus, insulin derived from microorganisms made by recombinant DNA techniques can be suitable for the galactosyl-insulin conjugates of the present invention.

The liver targeting component of the galactosyl-insulin conjugates of the present invention is the D-galactosyl group. As used herein, the term "D-galactosyl group" refers to a galactosyl glycoside capable of targeting the conjugate to the hepatocytes. The preferred D-galactosyl groups are D-galactosyl and N-acetyl-D-galactosylamine (2-acetamido-2-deoxy-D-galactosyl) glycosides. The hepatocytes have a high degree of affinity for N-acetyl-D-galactosylamine moieties which are especially preferred in the D-galactosyl group.

The D-galactosyl group is covalently bonded to insulin such that the conjugate formed retains significant biological activity. This conjugate can be represented structurally by the formula:

$$\left[ \text{Gal} - \text{L} \right]_n - \text{I}$$

wherein Gal is the D-galactosyl group; I is the insulin molecule; L is a linking group which covalently bonds Gal to I; and n is at least 1. Examples of suitable L groups are those which provide amides, amines, esters, ureas, thioureas, or ethers containing a covalent bond linking the D-galactosyl group to the insulin molecule.

- 9 -

Frequently, the Gal-L molecule is formed synthetically from a naturally existing material prior to attachment to the insulin molecule. For example, lactose and melibiose can be oxidized to lactobionic acid and melibionic acid, respectively. In addition, D-galactosyl groups can be attached to a variety of hydroxycarboxylic acids, mercaptocarboxylic acids or aminocarboxylic acids by O-, S- or N-glycosidic linkages, respectively, to form D-galactosyl-linking molecules. Examples of O-D-galactosyl-linking molecules include O-D-galactopyranosyl-(1→4)-D-gluconic acid (lactobionic acid), O-D-galactopyranosyl-(1→6)-D-gluconic acid (melibionic acid), O-D-galactosyl(1→2, 3, or 5)-D-gluconic acid, O-D-galactosyl-(1→2, 3, 4, 5 or 6)-galactonic acid, O-D-galactosyl-(1→2, 3, 4 or 5)-L-arabonic acid, O-D-galactosyl-(1→2, 3, 4 or 5)-D-xylonic acid, O-D-galactosyl-(1→2 or 3)-tartaric acid, O-D-galactosyl-(1→2)-malic acid, O-D-galactosyl-(1→2)-glycolic acid, O-D-galactosyl-(1→2)-lactic acid, O-D-galactosyl-(1→4)-butyric acid and O-D-galactosyl-(1→3)-citric acid.

Examples of S-D-galactosyl-linking molecules include S-D-galactosyl-(1→6)-D-gluconic acid, S-D-galactosyl-(1→4)-butyric acid, S-D-galactosyl-(1→3)-propanoic 'acid, S-D-galactosyl-(1→2) acetic acid and O-D-galactosyl-(1→4)-S-D-glucosyl-(1→6)-hexanoic acid.

Examples of N-D-galactosyl-linking molecules include O-β-D-galactosyl-(1→4)-N-D-glucosyl-(1→4)-butyric acid, O-D-galactosyl-(1→6)-N-D-glucopyranosyl-(1→3)-propanoic acid, N-D-galactosyl-(1→2)-acetic acid, and N-D-galactosyl-(1→5)-pentanoic acid.

For the preceding D-galactosyl-linking molecules, an N-acetyl-galactosaminyl group can be substituted for D-galactosyl.

The D-galactosyl-linking molecules are preferably covalently bonded to the amino groups of the insulin molecule by an amide linkage. Because insulin contains three such amino groups (terminal phenylalanine and glycine residues and an epsilon-amino group on a lysine residue), 1, 2 or 3 D-galactosyl groups can be covalently bonded by amide linkages. Thus, a mixture of conjugates can be formed wherein the mole ratio of D-galactosyl groups to insulin ranges from 1:1 to 3:1 (n being from 1 to 3). These mixtures, (mole ratio typically from about 1.3:1 to about 2.6:1) can be purified to obtain conjugates wherein n is from 1 to 2.

### B. Methods for Preparing Galactosyl-Insulin Conjugates

The galactosyl-insulin conjugates of the present invention can be prepared by certain conventional methods used in the synthesis of peptides. For example, a D-galactosyl-linking molecule, which contains a carboxylic acid group, can be linked to an amino group of insulin to form an amide bond. This method usually requires conversion of the carboxylic acid group to an activated form which reacts with the amino group. Acid chlorides, mixed anhydrides, acylimidazoles, carbodiimides, N-hydroxysuccinimide esters and the like can be used for this purpose. The N-hydroxysuccinimide esters are especially useful for the preparation of galactosyl-insulin conjugates because the reaction can be carried out in an aqueous medium under mild conditions of temperature and pH.

With regard to formation of the activated ester, the acid and a coupling agent such as N,N'-dicyclohexylcarbodiimide are dissolved in an aprotic solvent such as pyridine. This solvent system is preferably substantially free of water to prevent deactivation of the coupling agent and prior hydrolysis of the activated ester formed.

With regard to formation of the galactosyl-insulin conjugates, the activated ester is dissolved in water and then added in small increments (e.g. dropwise) to a saline solution containing the insulin. The particular mole ratio of D-galactosyl groups to insulin is especially dependent on the amount of activated ester used relative to the amount of insulin and the pH of the reaction mixture. Increasing the ratio of activated ester to insulin generally increases the ratio of D-galactosyl groups to insulin in the conjugates. At a pH of 7, the epsilon-amino group of lysine (pKa of about 10) is slowly acylated whereas the amino groups of glycine and phenylalamine are readily acylated (pKa's of 7.6 to 8.4); conjugates having a ratio of D-galactosyl groups to insulin of less than 2:1 are more likely. At a pH of 8.5, the epsilon-amino group of lysine is acylated to a greater degree; conjugates having a ratio of D-galactosyl groups to insulin of greater than 2:1 are more likely if a sufficient amount of activated ester is used.

Another method for preparing galactosyl-insulin conjugates of the present invention is by appropriate modification of the method described in Y.C. Lee, C.P. Stowell and M.J. Krantz, Biochemistry, 15 (18), 3956 (1976). In this method, cyanomethyl-1-thio-D-galactoside is converted to 2-imino-2-methoxyethyl-1-thio-D-galactoside with sodium methoxide or HCl in methanol. The 2-imino-2-methoxyethyl glycoside is reacted with the amino groups of insulin under slightly alkaline conditions (pH of about 8.5) to give a galactosyl-insulin conjugate coupled by an amine bond.

Another method for preparing the galactosyl-insulin conjugates of the present invention involves conversion of an O-aminoaryl-D-galactoside to the corresponding isothiocyanate. The isothiocyanate can be coupled with an amino group of insulin to form a thiourea-type galactosyl-insulin conjugate.

Another method for preparing galactosyl-insulin conjugates of the present invention is by conversion of an O-aminoaryl-D-galactoside to the corresponding diazonium salt and reacting the diazonium salt with insulin.

Another method for preparing galactosyl-insulin conjugates of the present invention is by formation of a bromoacetamide derivative of an amine-containing D-galactoside followed by coupling the bromoacetamide with an amino group(s) of insulin.

Another method for preparing galactosyl-insulin conjugates of the present invention is by treatment of a mixture of insulin and a disaccharide or oligosaccharide containing a nonreducing terminal D-galactosyl group with an alkali metal cyanoborohydride according to the method disclosed in Gray, Arch. Biochem. Biophys., 163, 426 (1974).

Still another method for preparing galactosyl-insulin conjugates of the present invention is to couple a D-galactosyl glycoside in which the aglycone moiety has an amino group with a carboxyl group of insulin using a carbodiimide coupling agent.

In this method the amino groups of insulin are preferably blocked to prevent coupling between insulin molecules.

C.   Use of Galactosyl-Insulin Conjugates in the Treatment of Diabetics

The galactosyl-insulin conjugates of the present invention are useful in the treatment of insulin-dependent diabetics, especially diabetic mammals, and in particular diabetic humans. Accordingly, the present invention provides a method for treating such diabetics by parenteral administration of the galactosyl-insulin conjugates to the diabetic in an amount effective to safely reduce the blood glucose level of the diabetic. As used herein, the term "parenteral administration" refers to introduction of the conjugates or compositions containing same into tissues of the body, other than by topical application or oral administration. Parenteral administration thus includes, without limitation, intrathecal, epidural, intramuscular, intravenous, intraperitoneal and subcutaneous administration. As used herein, the term "amount effective to safely reduce the blood glucose level" refers to an amount of the conjugates which is effective to provide a significant but safe reduction in blood glucose level of the diabetic at a reasonable benefit/risk ratio attendant with any medical treatment. Within the scope of sound medical judgment, the amount of the conjugates administered will vary with the severity of the diabetic condition, the duration of the treatment, the specific formulation employed, and like factors within the specific formulation employed, and like factors within the specific knowledge and expertise of the patient or attending physician. The galactosyl-insulin conjugates of the present invention are typically administered at the rate of from about 10 micro Units/kg/day to about 10 Units/kg/day, and preferably at the rate of from about 500 micro Units/kg/day to about 1 Unit/kg/day.

The galactosyl-insulin conjugates of the present invention are preferably formed into compositions for parenteral administration. These compositions comprise, or consist essentially of, the conjugates of the present invention and a pharmaceutically acceptable carrier(s). As used herein, the term "pharma-

- 13 -

ceutically acceptable carrier" denotes a liquid material which can be used in a pharmaceutical composition. Examples of materials which can serve as pharmaceutical carriers include pyrogen-free water, isotonic saline and phosphate buffer solutions. The pharmaceutical carrier or carriers are employed with the conjugates of the present invention so as to provide a conjugate concentration sufficient for a practical size to dosage relationship. Typically, the pharmaceutical carrier comprises at least about 99% by weight of the composition, and preferably at least about 99.99% by weight.

Preparation of Lactobionyl- and Melibionyl-Insulin Conjugates

The following embodiments relate to methods for preparing lactobionyl- and melibionyl-insulin conjugates and are illustrative of methods for making galactosyl-insulin conjugates according to the present invention.

Embodiment 1

A. Preparation of Lactobionate and Melibionate Ester of N-hydroxysuccinimide

2.0 g. of lactobionic acid dissolved in 10 ml. of pyridine was stirred with 1.20 g. of dicyclohexylcarbodiimide (DCC) at a temperature of 0°C for 0.5 hour. 0.64 g. of N-hydroxysuccinimide was added to this mixture which was then stirred for 20 hours at 3°C. This reacted mixture was filtered to remove dicyclohexylurea (DCU). The filtrate was evaporated to dryness on a rotary evaporator (bath temperature of 40°C) to provide a residue containing the lactobionate ester of N-hydroxysuccinimide which was stored at 3°C until used. The melibionate ester was obtained by the same procedure starting with melibionic acid.

B. Preparation of Lactobionyl- and Melibionyl-Insulin Conjugates

0.6 g. of the lactobionate ester of N-hydroxysuccinimide was dissolved in water. This aqueous solution was filtered to remove residual DCU. The filtrate was extracted three times with 15 ml. aliquots of methylene chloride to remove unreacted DCC. The aqueous layer containing the lactobionate ester was added dropwise over 30 minutes to 20 ml. of a stirred saline solution containing 6000 units of insulin at room temperature. During addition of the lactobionate ester, the saline solution was maintained at a pH of 7 by the addition of 0.5N sodium hydroxide. After all of the lactobionate ester had been added, the reaction mixture was allowed to stand for 1 hour at room temperature and then stored at 3°C. The reaction mixture was separated by gel filtration to obtain a mixture of lactobionyl-insulin conjugates.

The yield of the lactobionyl-insulin conjugates was 0.24 g. The conjugates had a lactobionyl-to-insulin mole ratio of 1.3:1 as determined by the phenol-sulfuric acid method of Dubois et al., Anal. Chem., 28, 350 (1956). This mixture of conjugates was separated by DEAE Sephadex column chromatography according to the method of Lindsay and Shall, Biochem. J., 121, 737-45 (1971), to obtain lactobionyl-insulin conjugates (n = 1) substituted at the N-terminal phenylalanine (B-1). When 2000 units of insulin was treated with 0.08 g. N-hydroxysuccinimide melibionate as described above, the yield of the melibionyl-insulin conjugates was 0.08 g. with a melibionyl-to-insulin mole ratio of 1.3:1.

### Embodiment 2

1.8 g. of the lactobionate ester of N-hydroxysuccinimide was reacted with a saline solution containing 6000 units of insulin as in Embodiment 1 except that the pH of the saline solution was maintained at 8.5. The yield of the conjugates was 0.26 g. The conjugates had a lactobionyl-to-insulin mole ratio of 2.6:1. This mixture of conjugates was separated by DEAE Sephadex column chromatography to obtain dilactobionyl-insulin conjugates (n = 2) substituted at N-terminal glycine (A-1) and phenylalanine (B-1).

### Biological Activity of Lactobionyl-Insulin
### Conjugates in Conscious Diabetic Dogs

A. Experimental Method

Diabetic dogs were maintained on daily doses of 2 units regular insulin plus 8 units Ultralente[R] insulin administered prior to feeding. Insulin doses were discontinued 48 hours prior to infusion of the lactobionyl-insulin conjugates. The dogs were placed in slings and 0.9% normal saline solution was infused into one cephalic vein of each dog at the rate of 0.5 ml./min. At 30 and 15 minutes prior to infusion of the conjugates, and at the start of infusion, a blood sample was obtained from the contralateral cephalic vein, transferred to a microhematocrit tube and centrifuged. The glucose content of the supernatant plasma from these blood samples was determined in duplicate with a Beckman Glucose Analyzer. The conjugates dissolved in 0.9% saline solution were then infused in place of the saline solution alone. Between 5 and 10 dogs were used in each experimental group. The conjugates were administered at a particular dose level to 1 or 2 dogs at the rate of 0.5 ml./min. At various time intervals, (see Tables below) blood samples were taken and the mg.% glucose determined. The relative percentage decrease in blood glucose due to the conjugates was calculated from the mg.% glucose at each sample time relative to the mg.% glucose at the start of infusion (zero time).

B. Results

1. Lactobionyl-Insulin Conjugate (phe B-1)

Dosage
(uU/kg/min)          Decrease in Blood Glucose (%)

min.

| | 0 | 15 | 30 | 60 | 90 | 120 |
|---|---|---|---|---|---|---|
| 0.4 | 0 | 4 | 4 | 5 | 10 | 11 |
| 4.0 | 0 | 4 | 7 | 10 | 16 | 16 |

2. Dilactobionyl-Insulin Conjugate (gly A-1 phe B-1)

Dosage
(uU/kg/min)          Decrease in Blood Glucose (%)

min.

| | 0 | 10 | 20 | 30 | 60 | 90 | 120 |
|---|---|---|---|---|---|---|---|
| 0.67 | 0 | -1 | 1 | -2 | 2 | 1 | -2 |
| 6.7 | 0 | 0 | 1 | 0 | 1 | 7 | 16 |
| 67 | 0 | -1 | -1 | 0 | 3 | 11 | 17 |

0119650

1. Galactosyl-insulin conjugates which are biologically active and are targeted to the liver, said conjugates being characterized in that they comprise:

insulin; and

at least one D-galactosyl group covalently bonded to said insulin.

2. The conjugates of Claim 1 characterized in that said D-galactosyl group is selected from the group consisting of D-galactosyl and N-acetyl-D-galactosylamine.

3. The conjugates of any of Claims 1-2 characterized in that they have the general formula $[Gal-L]_n-I$ wherein Gal is a D-galactosyl group; I is an insulin molecule; L is a linking group which covalently bonds Gal to I, and n is at least 1.

4. The conjugates of Claim 3 characterized in that Gal-L is an O-, S-, or N- D-galactosyl-glycoside, and n is from 1 to 3.

5. The conjugates of Claim 4 characterized in that D-galactosyl-glycoside is selected from the group consisting of lactobionyl and melibionyl.

6. The conjugates of any of Claims 3-5 characterized in that n is from 1 to 2.

7. A pharmaceutical composition for parenteral administration characterized in that it comprises the conjugates of any of Claims 1-6 and at least 99% by weight of a pharmaceutically acceptable carrier.

/jmc/EWG2/101